(19) Europäisches Patentamt
European Patent Office
Office européen des brevets

(11) **EP 4 116 982 A1**

(12) **EUROPEAN PATENT APPLICATION**
published in accordance with Art. 153(4) EPC

(43) Date of publication:
**11.01.2023 Bulletin 2023/02**

(51) International Patent Classification (IPC):
**G16B 30/10** (2019.01)  **G16B 40/10** (2019.01)

(21) Application number: **20928069.2**

(86) International application number:
**PCT/CN2020/090177**

(22) Date of filing: **14.05.2020**

(87) International publication number:
**WO 2021/196357 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.04.2020 CN 202010254696**

(71) Applicant: **Shanghai Zj Bio-tech Co., Ltd.**
**Pudong, Shanghai 201114 (CN)**

(72) Inventors:
• **JI, Cong**
**Shanghai 201114 (CN)**

• **SHAO, Junbin**
**Shanghai 201114 (CN)**
• **LIU, Yan**
**Shanghai 201114 (CN)**
• **QI, Xia**
**Shanghai 201114 (CN)**
• **JIN, Yudan**
**Shanghai 201114 (CN)**
• **LI, Qiteng**
**Shanghai 201114 (CN)**

(74) Representative: **Canzler & Bergmeier**
**Patentanwälte**
**Partnerschaft mbB**
**Despag-Straße 6**
**85055 Ingolstadt (DE)**

(54) **METHOD AND DEVICE FOR OBTAINING SPECIES-SPECIFIC CONSENSUS SEQUENCES OF MICROORGANISMS AND APPLICATION**

(57)     Provided is a method for obtaining species-specific consensus sequences of microorganisms, at least comprising the following steps: S100, searching for candidate consensus sequences: clustering specific sequences of various target strains belonging to the same strain on the basis of a clustering algorithm to obtain a plurality of candidate species-specific consensus sequences; and S200, verifying and obtaining species-specific consensus sequences of a first screening: determining whether the candidate species-specific consensus sequences meet the following conditions: 1) the plant seed coverage degree meets a preset value; 2) the effective copy number meets a preset value; and if the candidate species-specific consensus sequences meet all of the above conditions, then same are the species-specific consensus sequences. The method is highly sensitive; a repetitive sequence may be found in an incompletely assembled motif; obtained species-specific consensus sequences are accurate, and may be identified to a the subspecies level; the conservative property of identified consensus sequences is high, and the maximum value of the plant seed coverage is achieved as much as possible with the fewest consensus sequences; and all logic modules have multiple verifications, so the accuracy is high.

FIG. 1

**Description**

**Technical Field**

The present disclosure relates to the field of bioinformatics, and in particular, to a method and a device for obtaining species-specific consensus sequences of microorganisms and a use thereof.

**Background**

DNA concentrations of pathogenic microorganisms in biological samples are mostly very low and close to the detection limit. Traditional Polymerase Chain Reaction (PCR) or real-time PCR is often lack of detection sensitivity. Other methods such as two-step nested PCR may have better sensitivity. However, these methods are time-consuming, costly, and have poor accuracy. Therefore, it is important to improve the detection sensitivity. One way is to find a suitable template region when designing primers and probes. Usually, plasmids and 16S rRNA are used.

However, using plasmids for primer design would cause some problems: Not all microorganisms contain species-specific plasmids. Some microorganisms even have no plasmids. First of all, the species specificity of plasmid DNA is uncertain. The sequences on plasmids of some species are highly similar to those on plasmids of other species. Therefore, plasmid-based PCR tests are at a high risk of producing false positive or false negative results. Many clinical laboratories still need to use other PCR primer pairs for confirmatory experiments. Secondly, plasmids are not universal. Some species do not have plasmids, so it is not possible to use plasmids to detect the species, let alone to design primers on plasmids to improve the detection sensitivity. For example, it has been reported that about 5% of Neisseria gonorrhoeae strains cannot be detected since they lack plasmids.

Similarly, using rRNA gene regions as templates for PCR detection also has some problems: although rRNA genes exist in the genomes of all microbial species, and there are often multiple copies that can improve detection sensitivity. In fact, not all rRNA genes are specific. For example, there is only one copy of rRNA gene in mycobacterium tuberculosis H37Rv. In addition, some changes in rRNA gene sequence are not suitable for detection. For example, between closely related species or even between strains of different subtypes of the same species, rRNA genes cannot meet the requirements of species specificity or even sub-species specificity because the sequence of rRNA genes is too conservative.

On the other hand, if a microorganism with an unknown sequence causes an outbreak of an epidemic, the pathogenic microorganism database will be updated continuously, which may cause the original probe primer design to fail to cover the epidemic pathogenic microorganisms, thereby affecting the quality of nucleic acid detection reagents.

**Summary**

The present disclosure provides a method and a device for obtaining species-specific consensus sequences of microorganisms and a use thereof.

A first aspect of the present disclosure provides a method for obtaining species-specific consensus sequences of microorganisms, which includes at least the following operations:

S100, searching for candidate consensus sequences: clustering specific sequences of target strains belonging to the same species based on a clustering algorithm to obtain a plurality of candidate species-specific consensus sequences;

S200, verifying and obtaining primary-screened species-specific consensus sequences:

judging whether the candidate species-specific consensus sequences meet the following conditions:

3) a strain coverage rate meets a preset value;
4) an effective copy number meets a preset value;

if the candidate species-specific consensus sequences meet all the above conditions, determining that the candidate species-specific consensus sequences are species-specific consensus sequences;

$$\text{the strain coverage rate} = (\text{number of target strains with the candidate}$$

$$\text{species-specific consensus sequence} / \text{total number of target strains}) * 100\%;$$

the effective copy number is calculated according to formula (I):

$$\sum_{i=0}^{n} Ci * (\frac{Si}{Sall})$$

(I);

n is a total number of copy number gradients of the candidate species-specific consensus sequences;

Ci is the copy number corresponding to the i-th candidate species-specific consensus sequence;

Si is the number of strains with the i-th candidate species-specific consensus sequence;

Sail is a total number of the target strains.

A second aspect of the present disclosure provides a device for obtaining species-specific consensus sequences of microorganisms, which includes at least the following modules:

a candidate consensus sequence searching module, configured to obtain a plurality of candidate species-specific consensus sequences by clustering specific sequences of target strains belonging to the same species based on a clustering algorithm;

a primary-screened species-specific consensus sequence verifying and obtaining module, configured to judge whether the candidate species-specific consensus sequences meet the following conditions:

1) a strain coverage rate meets a preset value;

2) an effective copy number meets a preset value;

if the candidate species-specific consensus sequences meet all the above conditions, determining that the candidate species-specific consensus sequences are species-specific consensus sequences;

the strain coverage rate = (number of target strains with the candidate

species-specific consensus sequence / total number of target strains) * 100%;

the effective copy number is calculated according to formula (I):

$$\sum_{i=0}^{n} Ci * (\frac{Si}{Sall})$$

(I);

n is a total number of copy number gradients of the candidate species-specific consensus sequences;

Ci is the copy number corresponding to the i-th candidate species-specific consensus sequence;

Si is the number of strains with the i-th candidate species-specific consensus sequence;

Sail is a total number of the target strains.

A third aspect of the present disclosure provides a computer readable storage medium, which stores a computer program. When executed by a processor, the program implements the above-mentioned method for obtaining species-specific consensus sequences of microorganisms.

A fourth aspect of the present disclosure provides a computer processing device, including a processor and the above-mentioned computer readable storage medium. The processor executes the computer program on the computer readable storage medium to implement the operations of the above-mentioned method for obtaining species-specific consensus sequences of microorganisms.

A fifth aspect of the present disclosure provides an electronic terminal, including a processor, a memory and a communicator; the memory stores a computer program, the communicator communicates with an external device, and the processor executes the computer program stored in the memory, so that the terminal executes the above-mentioned method for obtaining species-specific consensus sequences of microorganisms.

A sixth aspect of the present disclosure provides a use of the above-mentioned method for obtaining species-specific consensus sequences of microorganisms, the above-mentioned device for obtaining species-specific consensus sequences of microorganisms, the above-mentioned computer readable storage medium, the above-mentioned computer processing device or the above-mentioned electronic terminal for screening template sequences in nucleotide amplification.

A seventh aspect of the present disclosure provides a method for identifying microbial species, including: identifying whether the target strain contains a species-specific consensus sequence by means of amplification; the species-specific consensus sequence is obtained by the above-mentioned method for obtaining species-specific consensus sequences of microorganisms, the above-mentioned device for obtaining species-specific consensus sequences of microorganisms, the above-mentioned computer readable storage medium, the above-mentioned computer processing device or the above-mentioned electronic terminal.

As described above, the method and the device for obtaining species-specific consensus sequences of microorganisms and the use thereof according to the present disclosure have the following beneficial effects:

the method is high in sensitivity, and an undiscovered multi-copy region can be identified; a repetitive sequence can be found in an incompletely assembled sequence motif; the obtained species-specific consensus sequences are accurate, and the subspecies level can be identified; However, if conservative, and the maximum value of the strain coverage rate is achieved as much as possible with the least consensus sequences; all the logic modules have multiple verifications, so that the accuracy is high. Users may select a suitable calculation scheme (i.e., giving preference to multicopy or specificity) according to different detection objects. A detection device designed with quantitative PCR primers and probes for systematic and automated detection of pathogenic microorganisms in biological samples may cover all pathogenic microorganisms, including bacteria, virus, fungi, amoebas, cryptosporidia, flagellates, microsporidia, piroplasma, plasmodia, toxoplasmas, trichomonas and kinetoplastids. Users may select different configuration parameters depending on the purpose of a project, the configuration parameters mainly include: name of workflow, target species, comparison species, uploading of local fasta files, length of target fragment, species specificity (similarity to other species), similarity of repeated regions, strain distribution of the target fragment, filtering of the host sequence, priority scheme (prioritizing multi-copy regions vs. prioritizing specific regions), calculation of similarity of target strain and similarity alarm threshold, and primer probe design parameters.

**Brief Description of the Drawings**

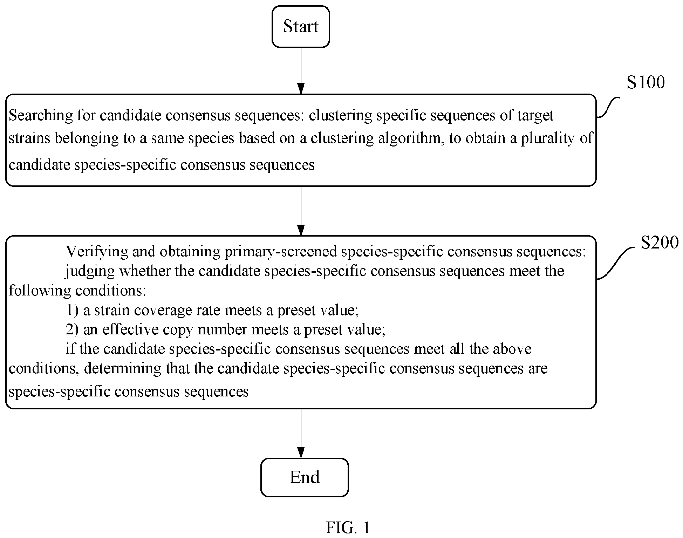

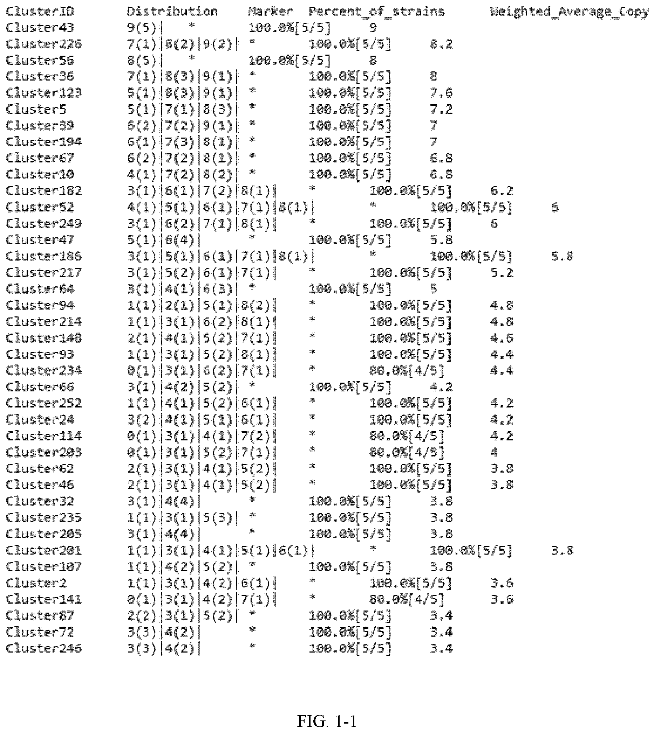

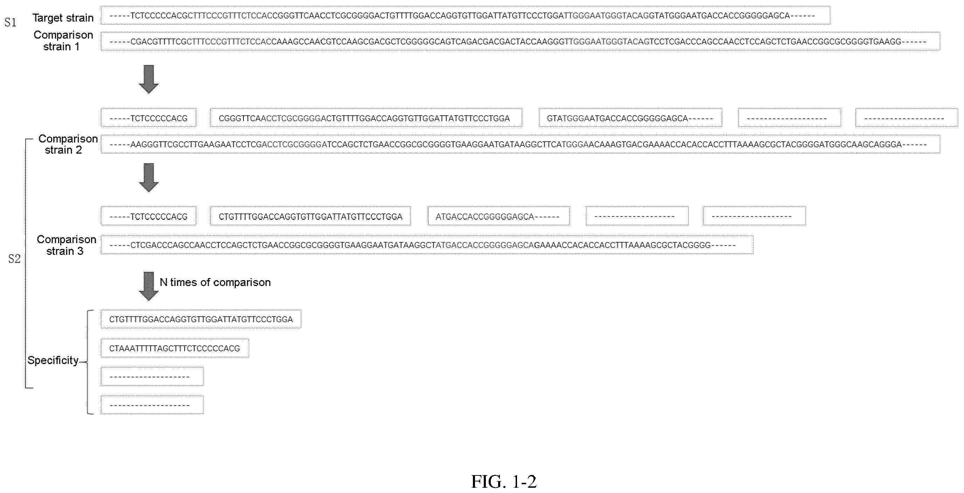

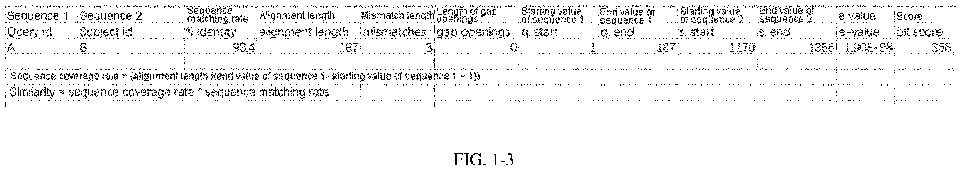

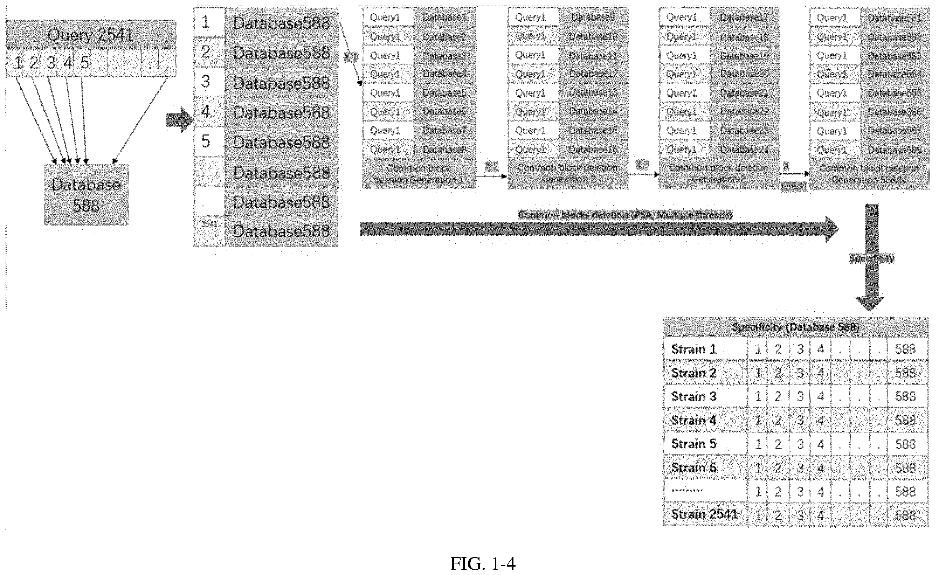

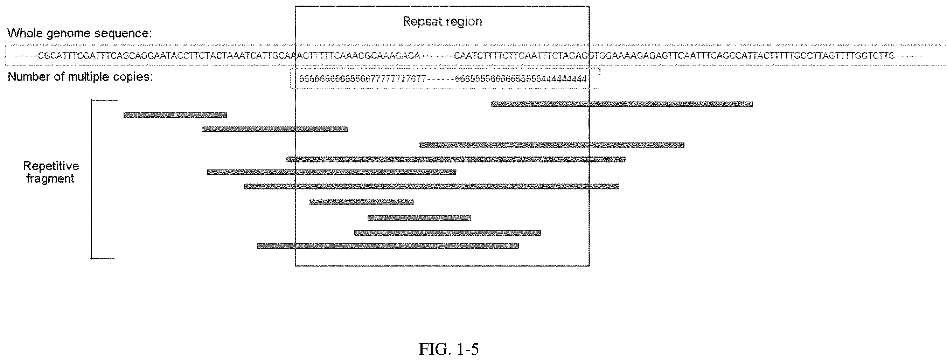

FIG. 1 is a flow chart of a method according to an embodiment of the present disclosure.

FIG. 1-1 is a schematic diagram of regions of candidate species-specific consensus sequences.

FIG. 1-2 is a schematic diagram showing a sequence of a method for obtaining a specific region according to an embodiment of the present disclosure.

FIG. 1-3 is a graph showing calculation results of a coverage rate and sequence matching rate of compared sequences.

FIG. 1-4 is a schematic diagram showing comparing the first-round cut fragment $T_n$ with whole genome sequences of the remaining comparison strains by group iteration in a method for obtaining a specific region according to the present disclosure.

FIG. 1-5 is a schematic diagram showing a sequence of a method for obtaining a specific region according to an embodiment of the present disclosure.

FIG. 2 is a schematic diagram of a device according to an embodiment of the present disclosure.

FIG. 3 is a schematic diagram of an electronic terminal according to an embodiment of the present disclosure.

**Detailed Description of the Preferred Embodiments**

The embodiments of the present disclosure will be described below. Those skilled in the art can be easily understood other advantages and effects of the present disclosure according to contents disclosed by the specification. The present disclosure may also be implemented or applied through other different specific implementation modes. Various modifications or changes may be made to all details in the specification based on different points of view and applications without departing from the spirit of the present disclosure.

In addition, it should be understood that one or more method operations mentioned in the present disclosure are not exclusive of other method operations that may exist before or after the combined operations or that other method operations may be inserted between these explicitly mentioned operations, unless otherwise stated. It should also be understood that the combined connection relationship between one or more operations mentioned in the present disclosure does not exclude that there may be other operations before or after the combined operations or that other operations may be inserted between these explicitly mentioned operations, unless otherwise stated. Moreover, unless otherwise stated, the numbering of each method step is only a convenient tool for identifying each method step, and is not intended to limit the order of each method step or to limit the scope of the present disclosure. The change or adjustment of the relative relationship shall also be regarded as the scope in which the present disclosure may be implemented without substantially changing the technical content.

Please refer to FIGs. 1-3. It needs to be stated that the drawings provided in the following embodiments are just used for schematically describing the basic concept of the present disclosure, thus only illustrating components only related to the present disclosure and are not drawn according to the numbers, shapes and sizes of components during actual implementation, the configuration, number and scale of each components during actual implementation thereof may be freely changed, and the component layout configuration thereof may be more complicated.

As shown in FIG. 1, a method for obtaining species-specific consensus sequences of microorganisms according to this embodiment includes the following operations:

S100, searching for candidate consensus sequences: clustering specific sequences of target strains belonging to the same species based on a clustering algorithm to obtain a plurality of candidate species-specific consensus sequences;

S200, verifying and obtaining the primary-screened species-specific consensus sequences:

judging whether the candidate species-specific consensus sequences meet the following conditions:

1) a strain coverage rate meets a preset value;
2) an effective copy number meets a preset value;

if the candidate species-specific consensus sequences meet all the above conditions, determining that the candidate species-specific consensus sequences are species-specific consensus sequences;

$$\text{strain coverage rate} = (\text{number of target strains with the candidate}$$

$$\text{species-specific consensus sequence / total number of target strains}) * 100\%;$$

the effective copy number is calculated according to formula (I):

$$\sum_{i=0}^{n} Ci * (\frac{Si}{Sall}) \qquad (I);$$

n is the total number of copy number gradients of the candidate species-specific consensus sequence. n may be obtained by calculating the copy number gradients after obtaining the copy numbers of the candidate species-specific consensus sequence in each strain;

Ci is the copy number corresponding to the i-th candidate species-specific consensus sequence;

Si is the number of strains with the i-th candidate species-specific consensus sequence;

Sall is a total number of the target strains.

The preset value of strain coverage rate may be determined according to needs. The higher the preset value, the greater the number of target strains covered by the screened species-specific consensus sequence, and the more representative they will be. Most preferably, the preset value of strain coverage rate is 100%. However, if the preset value of strain coverage rate actually cannot reach 100%, it may be reduced in order, such as 100%, 99%, 98%, 97%, or 96%.

The preset value of the effective copy number may be determined as needed. The preset value of the effective copy number preferably exceeds 1, for example, the preset value of the effective copy number may be 2, 3, 4, 10, 20, etc.

Formula (I) refers to the summation of Ci (Si/Sall), where i ranges from Cmin to Cmax, and the number of i is n. Cmin is the minimum copy number of all candidate species-specific consensus sequences. Cmax is the maximum copy number of all candidate species-specific consensus sequences.

The candidate species-specific consensus sequences may be compared to the whole genomes of all target strains, to calculate the strain coverage rate and effective copy number of the candidate species-specific consensus sequence.

Furthermore, the number of copies of a candidate species-specific consensus sequence on the whole genome of a target strain is calculated by re-comparing the candidate species-specific consensus sequence to the whole genome sequence of each target strain. By analogy, the number of copies of the candidate species-specific consensus sequence on the whole genome of all target strains is calculated, and Sall copy number values are obtained. Copy number values are arranged from small to large, and the number of covered strains correspond to each copy number value is calculated.

Specifically, taking FIG. 1-1 as an example, the 5 target strains all contain the region cluster 43 of the candidate species-specific consensus sequence, and the strain coverage rate reaches 100% (5/5). The copy number distribution 9 (5) means that there are 5 strains with a copy number of 9, and the copy number gradient is 1. It can be seen that n=1, Cmin and Cmax are both 9, and Si and Sall are both 5. By substituting the above into formula (I), the effective copy number = 9*(1/1) = 9. Therefore, the effective copy number of region cluster43 is 9.

As another example, in FIG. 1-1, the 5 target strains all contain the region cluster226 of the candidate species-specific consensus sequence, and the strain coverage rate reaches 100% (5/5). The copy number distribution 7(1)/8(2)/9(2) means that there are one strain with a copy number of 7, two strains with a copy number of 8, and two strains with a copy number of 9, the copy number has 3 gradients. It can be seen that n=3, Cmin and Cmax are 7 and 9, respectively, C1 is 7, C2 is 8, C3 is 9, S1=1, S2=2, S3=2, Sall=5. By substituting the above into formula (I), the effective copy number =7*(1/5)+8*(2/5)+9*(2/5)=8.2. Therefore, the effective copy number of region cluster226 is 8.2.

In operation S100, after clustering, similar specific multi-copy sequences form a set, and each set corresponds to a consensus sequence.

The clustering algorithm used in clustering can cluster all the specific sequences. According to the principle of sequence similarity, the sequence that best represents the group in different groups is selected as the consensus sequence, and the consensus sequence is the closest to all the sequences in the group.

The specific sequence refers to the target fragments belonging to the same target strain, and the region where the target fragments are located is a specific region of the target strain. The specific region may be a specific single-copy region or a specific multi-copy region. As the amplification based on a multi-copy region has stronger operability, a specific multi-copy region is preferred. A target strain may have multiple specific multi-copy sequences.

The method for obtaining a specific region includes the following operations:

S110, respectively comparing a microorganism target fragment with whole genome sequences of one or more comparison strains one-to-one, and removing fragments of which the similarity exceeds a preset value, to obtain a plurality of residual fragments as first-round cut fragments $T_1$-$T_n$, n is an integer greater than or equal to 1;
S120, respectively comparing the first-round cut fragments $T_1$-$T_n$ with whole genome sequences of remaining comparison strains, and removing fragments of which a similarity exceeds the preset value, to obtain a collection of residual cut fragments as a candidate specific region of the microorganism target fragment; and
S130, verifying and obtaining a specific region: determining whether the candidate specific region meets the following requirements:

1) searching in public databases to find whether there are other species of which a similarity to the candidate specific region is greater than the preset value;
2) respectively comparing the candidate specific region with whole genome sequences of the comparison strains and a whole genome sequence of a host of a source strain of the microorganism target fragment, to find whether there are fragments with a similarity greater than the preset value;

if the candidate specific region does not meet the above requirements, the candidate specific region is a specific region of the microorganism target fragment.

The method of the present disclosure is capable of distinguishing whether the source strain of the microorganism target fragment and a comparison strain belong to the same species or subspecies.

In the above operations, the similarity refers to a product of a coverage rate and a matching rate of the microorganism target fragment.

$$\text{The coverage rate} = (\text{length of similar sequence fragment}/(\text{end value of the}$$

$$\text{microorganism target fragment} - \text{starting value of the microorganism target fragment}$$

$$+ 1))\%;$$

The matching rate refers to the identity value when the microorganism target fragment is compared with the comparison strain. The identity value of the two compared sequences may be obtained by software such as needle, water or blat.

The length of similar sequences refers to the number of bases that the matched fragment occupies in the target fragment when two sequences are compared, that is, the length of the matched fragment.

The preset value of the similarity may be determined as needed. The higher the preset value of the similarity, the fewer fragments will be removed. The recommended preset value of the similarity should exceed 95%, such as 96%, 97%, 98%, 99% or 100%.

The specific sequence is shown in FIG. 1-2, and the light-colored bases represent sequence fragments of which the similarity exceeds the preset value.

The coverage rate and matching rate of microorganism target fragments may be calculated by software such as needle, water or blat.

For example, a calculation result is shown in FIG. 1-3. Sequence A is a microorganism target fragment, sequence B is the comparison strain 1. Sequences A and B are compared.

$$\text{Coverage rate of sequence A} = (187/(187-1+1))*100\% = 100\%$$

The matching rate of sequence A and sequence B is equal to 98.4%.

Then the similarity between A and B=100%*98.4%=98.4%.

The microorganism target fragment and the comparison strains in operation S110 are all derived from public databases, which are mainly selected from NCBI (https://www.ncbi.nlm.nih.gov).

The method further comprises: Sill, comparing selected adjacent microorganism target fragments one-to-one; if the similarity after comparison is lower than the preset value, issuing an alarm and displaying screening conditions corresponding to a target strain. Abnormal data and redundant data caused by human errors can be filtered.

The microorganism target fragment in operation S110 may be a whole genome of a microorganism or a gene fragment of a microorganism.

In operation S120, in order to speed up the comparison, in a preferred embodiment, the first-round cut fragments $T_1$-$T_n$ are respectively compared with whole genome sequences of the remaining comparison strains by group iteration.

Specifically, as shown in FIG. 1-4, the first-round cut fragment $T_n$ being compared with whole genome sequences of the remaining comparison strains by group iteration includes the following operations:

S121, dividing the remaining comparison strains into P groups, each group including a plurality of comparison strains;
S122, simultaneously comparing the first-round cut fragment $T_n$ with the whole genome sequences of the comparison strains in the first group one-to-one, and removing fragments of which the similarity exceeds the preset value, to obtain a plurality of residual fragments as the first-round candidate sequence library of the first-round cut fragment $T_n$;
S123, simultaneously comparing the previous-round candidate sequence library of the first-round cut fragment $T_n$ with the whole genome sequences of the comparison strains in the nest group one-to-one, and removing fragments of which the similarity exceeds the preset value, to obtain a plurality of residual fragments as the next-round candidate sequence library of the first-round cut fragment $T_n$; repeating operation S122 from the first-round candidate sequence library until a P-th-round candidate sequence library is obtained as the candidate specific sequence library of the first-round cut fragment $T_n$;
a collection of all the candidate specific sequence libraries of the first-round cut fragments is the candidate specific region.

In order to avoid multi-thread blocking, the number of comparison strains contained in a comparison strain group should be set according to the hardware configuration of the computing environment. The number may be the number of threads set according to the total configuration of the operating environment. Generally, the number of threads may be 1-50. Specifically, the number of threads may be 1-4, 4-8, 8-10, 10-20, or 20-50. Preferably, the number of threads is 4. In the embodiment shown in FIG. 1-2, the number of threads is 8.

For example, as shown in FIG. 1-4, the target sequence contains 2541 microorganism target fragments, the number of the comparison strains is 588, m=8. First, simultaneously comparing the microorganism target fragment 1 with the sequences 1-8 in the 588 comparison strains, performing the first-round cutting to remove the matched sequences, and obtaining the first-round specific sequence library after a comprehensive summary; then, simultaneously comparing the first-round specific sequence library with the sequences 9-16 in the 588 comparison strains, performing the second-round cutting to remove the matched sequences, and obtaining the second-round specific sequence library after a comprehensive summary; then, simultaneously comparing the second-round specific sequence library with the sequences 17-24 in the 588 comparison strains, performing the third-round cutting to remove the matched sequences, and obtaining the third-round specific sequence library after a comprehensive summary; ..., performing sequentially, until the 73th-round specific sequence library is simultaneously compared with the sequences 585-588 in the 588 comparison strains, the matched sequences are removed by performing the 74th-round cutting, and the 74th-round specific sequence library, i.e., the specific sequence library of the target fragment 1, is obtained after a comprehensive summary.

Secondly, simultaneously comparing the microorganism target fragment 2 in the target sequence with the sequences 1-8 in the 588 comparison strains, performing the first-round cutting to remove the matched sequences, and obtaining the first-round specific sequence library after a comprehensive summary; then, simultaneously comparing the first-round specific sequence library with the sequences 9-16 in the 588 comparison strains, performing the second-round cutting to remove the matched sequences, and obtaining the second-round specific sequence library after a comprehensive summary; then, simultaneously comparing the second-round specific sequence library with the sequences 17-24 in the 588 comparison strains, performing the third-round cutting to remove the matched sequences, and obtaining the third-round specific sequence library after a comprehensive summary; ..., performing sequentially, until the 73th-round specific sequence library is simultaneously compared with the sequences 585-588 in the 588 comparison strains, the matched sequences are removed by performing the 74th-round cutting, and the 74th-round specific sequence library, i.e., the specific sequence library of the target fragment 2, is obtained after a comprehensive summary.

Performing sequentially, until the comparison of the microorganism target fragment 2541 in the target sequence and the 588 comparison strains are completed. The cut fragments obtained are the candidate specific regions of the microorganism target fragments.

In a preferred embodiment, the operation S120 further includes:
performing operations S110 and S120 to obtain candidate specific regions of each microorganism target fragment in the target sequence, taking a collection of the candidate specific regions of each microorganism target fragment as candidate specific regions of the target sequence.

The target sequence may include multiple target fragments. The multiple target fragments may be fragments obtained by screening from the genome of microorganisms through other screening operations, for example, multi-copy fragments of specific microorganisms.

In operation S130, the public databases are mainly selected from NCBI (https://www.ncbi.nlm.nih.gov). The algorithm for searching in the public database may be the blast algorithm.

Further, before performing operations S110, S120 and S130, the cutting size is set according to the hardware configuration of the computing environment, and the data to be calculated is cut in units. Specifically, in operation S110, the data to be calculated is the target fragments. In operation S120, the data to be calculated is the current-round specific sequence library after removing the matched sequences in each iteration. In operation S130, the data to be calculated is the candidate specific region.

After cutting in units, the number of units * the configuration required to run a unit file cannot exceed the total configuration of the operating environment.

Cutting in units refers to dividing the total number of the to-be-cut sequences by the number of threads, and m is recorded as the number of units after cutting in units. Each thread runs the same number of computing tasks in a multi-thread operating environment to ensure efficient computing under optimal performance conditions.

The method for obtaining a multi-copy region includes the following operations:

S140, searching for a candidate multi-copy region: performing an internal alignment on a microorganism target fragment, and searching for a region corresponding to a to-be-detected sequence of which a similarity meets a preset value as a candidate multi-copy region, the similarity being a product of a coverage rate and a matching rate of the to-be-detected sequence;

S150, verifying and obtaining a multi-copy region: obtaining a median value of copy numbers of the candidate multi-copy region; if the median value of the copy numbers of the candidate multi-copy region is greater than 1, the candidate multi-copy region is recorded as a multi-copy region.

The preset value of the similarity may be adjusted as needed. The recommended preset value of the similarity should exceed 80%, such as 85%, 90%, 95%, 96%, 97%, 98%, 99%, or 100%.

$$\text{The coverage rate} = (\text{length of similar sequence}/(\text{end value of the}$$

$$\text{to-be-detected sequence - starting value of the to-be-detected sequence} + 1))\%.$$

The matching rate refers to the identity value when the to-be-detected sequence is aligned with another sequence. The identity value of the two compared sequences may be obtained by software such as needle, water or blat.

The length of similar sequences refers to the number of bases that the matched fragment occupies in the to-be-detected sequence when the to-be-detected sequence is aligned with another sequence, that is, the length of the matched fragment.

For example, the data situation of a to-be-detected sequence corresponding to a candidate multi-copy region is shown in FIG. 1-1.

Sequence A is the to-be-detected sequence; when sequence A is aligned with sequence B, the length of the matched fragment is 187, the starting value (i.e., the starting position) of sequence A is 1, and the end value (i.e., the ending position) is 187, then:

$$\text{Coverage rate of sequence } A = (187/(187-1+1))*100\%=100\%.$$

The matching rate of sequence A and sequence B corresponds to an identity of 98.4%.

Then the similarity between A and B=100%*98.4%=98.4%. The similarity preset value is 80%. The similarity between A and B satisfies the preset value. Therefore, A and B serve as candidate multi-copy regions.

The positions of the bases between the two to-be-aligned sequences do not cross (that is, the two aligned sequences are separated in the microorganism target fragment, and there is no overlapping part). The aligned sequence pair with regional overlapping may be removed before or after the alignment to obtain the similarity value. For example, as shown in FIG. 1-3, the positions of the bases in sequence B will not appear between 1-187 if the position of sequence A is 1-187. After the coverage rate and match rate are calculated, the uniq function may be used for de-duplication.

In operation S150, the obtaining of the median value of the copy numbers of the candidate multi-copy region includes: determining the position of each candidate multi-copy region on the microorganism target fragment, obtaining the number of other candidate multi-copy regions covering the position of each base of the to-be-verified candidate multi-copy region, and calculating the median value of the copy numbers of the to-be-verified candidate multi-copy region. The above-mentioned other candidate multi-copy regions refer to candidate multi-copy regions other than the to-be-verified candidate multi-copy region.

Specifically, for example, as shown in FIG. 1-5, the first row represents the sequence of the microorganism target fragment. In the sequence of the microorganism target fragment, the fragment within the frame is the to-be-verified candidate multi-copy region. The number in the second row is the number of multiple copies corresponding to each base in the to-be-verified candidate multi-copy region. The gray fragments in the figure represent the candidate multi-copy regions other than the to-be-verified candidate multi-copy region (hereinafter referred to as repetitive fragments). From the left to the right, the first base A in the first row of the frame appears in 5 repetitive fragments (that is, covered by 5 repetitive fragments). Therefore, it is considered that the number of repetitive fragments corresponding to the position of the first base A is 5, then the number of multiple copies at this position is 5. Take the last base G in the frame in the figure as another example, the number of repetitive fragments corresponding to the position of the last base G is 4, that is, the number of multiple copies at this position is 4. By analogy, the number of repetitive fragments covering the position of each base of the to-be-verified candidate multi-copy region is counted. For statistical results, see the number of multiple copies in the second row in the figure. By combining the values of the copy numbers of each position, the median value of the copy numbers of the candidate multi-copy regions can be obtained. The median value refers to the variable value positioned in the middle of a variable series that is formed by arranging the variable values in the statistical population in order of value size.

The repetitive fragment refers to a candidate multi-copy region other than a to-be-verified candidate multi-copy region, and the position of each repetitive fragment corresponds to the original position of the repetitive fragment in the whole genome.

Further, in operation S140, the microorganism target fragment may be a chain or multiple incomplete motifs.

When the microorganism target fragment includes multiple incomplete motifs, the motifs are connected together before searching for candidate multi-copy regions. There is no specific restriction on the order in which the motifs are connected together. The motifs may be connected in any order. For example, the motifs may be connected into a chain in random order. If a region where the similarity meets the preset value contains different motifs, the region is cut based on the original motif connection point and divided into two regions, to determine whether the two regions are candidate multi-copy regions, respectively.

The motifs may be connected in a random way.

The microorganism target fragment being multiple incomplete motifs means that part of the sequence of the microorganism target fragment is not a continuous single sequence, but is composed of multiple motifs of different sizes. The motif is caused by incomplete splicing of short read lengths under the existing second-generation sequencing conditions.

The method of the present disclosure is not limited to whether there is a whole genome sequence. Operational tasks can be submitted by providing the names of the target strain and comparison strain or by uploading sequence files locally. In terms of detection scope, the method for identifying multi-copy regions in microorganism target fragments may cover all pathogenic microorganisms, including but not limited to bacteria, virus, fungi, amoebas, cryptosporidia, flagellates, microsporidia, piroplasma, plasmodia, toxoplasmas, trichomonas and kinetoplastids.

In a preferred embodiment, in operation S150, a 95% confidence interval of the copy numbers of the candidate multi-copy region may be calculated. The confidence interval refers to the estimated interval of the overall parameter constructed by the sample statistics, that is, the interval estimation of the overall copy numbers of the target region. The confidence interval reflects the degree to which the true value of the copy numbers of the target region has a certain probability to fall around the measurement result. The confidence interval gives the credibility of the measured value of the measured parameter.

When calculating the 95% confidence interval of the copy numbers of the candidate multi-copy region, the base number of the candidate multi-copy region serves as the sample number, and the copy number value corresponding to each base in the candidate multi-copy region serves as the sample value.

As shown in FIG. 1-5, in the multi-copy target region with a length of 500 bp, each base corresponds to one copy number value, then a set of 500 copy number values in total are located in the multi-copy target region.

In addition to the median value of the copy numbers mentioned above, the present disclosure uses the 95% confidence interval of these 500 copy number values to measure the interval estimation of the overall copy numbers of the multi-copy target region when the significance level is 0.05 and the confidence level is 95%. When the confidence level is the same, the more samples, the narrower the confidence interval and the closer to the mean value.

The microorganism target fragment may be a whole genome of a microorganism or a gene fragment of a microorganism.

The mechanism to obtain the multi-copy region is that, under normal circumstances, the median value and 95% confidence interval representing these 500 copy number values can reflect the real condition of the candidate multi-copy region. In addition to further verifying the multiple copies, the design of the module can also exclude some special cases. For example, if only 5 bases in the 500-bp candidate multi-copy region have a copy number of 1000, and the remaining 495 bases have a copy number of 1, then in this case, the median value of the copy numbers is 1, but the mean value is 10.99, and the 95% confidence interval ranges from 2.25 to 19.73. Obviously, although the mean value indicates multiple copies, the median value is no longer within the 95% confidence interval. Therefore, the candidate multi-copy region cannot be judged as a multi-copy region.

In a further preferred technical scheme, the method further includes the following operations:

S300, obtaining the candidate probes and primers by designing the probes and primers for the primary-screened species-specific consensus sequence according to the design rule of probes and primers; aligning the sequence of the candidate probes and primers to the whole genome of all target strains, calculating the strain coverage rate corresponding to the sequence of each probes and primers, screening out the candidate probes and primers of which the strain coverage rate meets a preset value, and taking the primary-screened species-specific consensus sequence corresponding to the screened candidate probes and primers as the final species-specific consensus sequence.

In an embodiment, the method further includes the following operations:

S400, if none of the strain coverage rates of the candidate consensus sequences in operation S200 reaches the preset value, combining the candidate consensus sequences, screening out a combination with a strain coverage rate reaching the preset value and having the least consensus sequence, taking the screened combination as the candidate consensus sequence, verifying and obtaining the primary-screened species-specific consensus sequences by S200.

In another embodiment, the method further includes the following operations:

S500, if none of the strain coverage rates of the candidate probes and primers in operation S300 reaches the preset value, combining the primary-screened species-specific consensus sequences, screening out a combination with a strain coverage rate reaching the preset value and having the least consensus sequence, taking the screened combination as the candidate consensus sequence, verifying and obtaining the primary-screened species-specific consensus sequences by S200.

In operations S400 and S500, the combination may be performed according to the number of consensus sequences from low to high for selection.

Specifically, two consensus sequences are combined first. Although there is no single consensus sequence that can cover all the strains, it may be possible to find two consensus sequences, where the sum of the strain coverage rates of the two consensus sequences is greater than or equal to the preset value of the strain coverage rate. If there are such two consensus sequences, the two consensus sequences are recorded in the result; if not, three consensus sequences are combined. That is, although there is no single consensus sequence or two consensus sequences that can meet the preset value of strain coverage rate, it may be possible to find three consensus sequences, where the sum of the strain coverage rates of the three consensus sequences is greater than or equal to the preset value of the strain coverage rate. If there are such three consensus sequences, the three consensus sequences are recorded in the result; if not, four consensus sequences are combined. By analogy, infinite number of consensus sequences may be combined, until a consensus sequence combination which can meet the preset value of the total strain coverage rate is found and recorded in the result.

In order to ensure the continuous update of the biomarker database, on the one hand, the latest data may be re-calculated by re-submitting the operational tasks. On the other hand, a sequence update coverage rate module may be used to verify the coverage rate of existing biomarkers in the updated sequence data set. When the number of target strains is updated, the original candidate probes and primers is aligned to the updated whole genome of the target strain. The coverage rate is calculated, and whether the original candidate probes and primers can cover the updated target strain is verified.

The species-specific consensus sequence screened by the method of the present disclosure can simultaneously meet multiple conditions such as specificity, sensitivity and conservation.

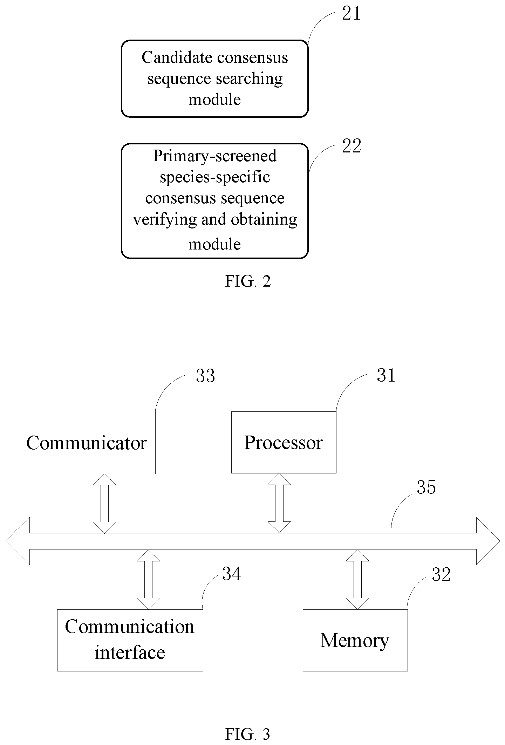

As shown in FIG. 2, the device for obtaining species-specific consensus sequences of microorganisms according to an embodiment of the present disclosure includes at least the following modules: a candidate consensus sequence searching module and a primary-screened species-specific consensus sequence verifying and obtaining module.

The candidate consensus sequence searching module obtains a plurality of candidate species-specific consensus sequences by clustering specific sequences of target strains belonging to a same species based on a clustering algorithm.

The primary-screened species-specific consensus sequence verifying and obtaining module judges whether the candidate species-specific consensus sequences meet the following conditions:

1) a strain coverage rate meets a preset value;
2) an effective copy number meets a preset value;

if the candidate species-specific consensus sequences meet all the above conditions, determining that the candidate species-specific consensus sequences are species-specific consensus sequences;

$$\text{the strain coverage rate} = (\text{number of target strains with the candidate}$$

$$\text{species-specific consensus sequence} / \text{total number of target strains}) * 100\%;$$

the effective copy number is calculated according to formula (I):

$$\sum_{i=0}^{n} Ci * (\frac{Si}{Sall}) \qquad (I);$$

n is a total number of copy number gradients of the candidate species-specific consensus sequences;
Ci is the copy number corresponding to the i-th candidate species-specific consensus sequence;
Si is the number of strains with the i-th candidate species-specific consensus sequence;

Sall is a total number of the target strains.

The specific sequence refers to the target fragments belonging to the same target strain, and the region where the target fragments are located is a specific region of the target strain.

The specific region is a specific multi-copy region.

The device may further include a first-round cut fragment obtaining module, a candidate specific region obtaining module, and a specific region verifying and obtaining module for obtaining specific regions.

The first-round cut fragment obtaining module respectively compares a microorganism target fragment with whole genome sequences of one or more comparison strains one-to-one, and removes fragments of which the similarity exceeds a preset value, to obtain a plurality of residual fragments as first-round cut fragments $T_1$-$T_n$, n is an integer great than or equal to 1.

The candidate specific region obtaining module respectively compares the first-round cut fragments $T_1$-$T_n$ with whole genome sequences of remaining comparison strains, and removes fragments of which the similarity exceeds the preset value, to obtain a collection of residual cut fragments as a candidate specific region of the microorganism target fragment.

The specific region verifying and obtaining module determines whether the candidate specific region meets the following requirements:

1) public databases are searched in to find whether there are other species of which a similarity to the candidate specific region is greater than the preset value;
2) the candidate specific region is compared with whole genome sequences of the comparison strains and a whole genome sequence of a host of a source strain of the microorganism target fragment respectively, to find whether there are fragments with a similarity greater than the preset value;

if the candidate specific region does not meet the above requirements, the candidate specific region is a specific region of the microorganism target fragment.

The device of the present disclosure is capable of distinguishing whether the source strain of the microorganism target fragment and the comparison strain belong to the same species or subspecies.

The similarity refers to a product of a coverage rate and a matching rate of the microorganism target fragment, and the coverage rate = (length of similar sequence fragment/(end value of the microorganism target fragment - starting value of the microorganism target fragment + 1))%.

The preset value of similarity exceeds 80%.

Positions of bases between two to-be-aligned sequences do not cross.

Optionally, the first-round cut fragment obtaining module further includes the following submodules: a raw data similarity comparison submodule, to compare the selected adjacent microorganism target fragments in pairs; if the similarity after comparison is lower than the preset value, an alarm is issued and the screening conditions corresponding to the target strain are displayed.

In the candidate specific region obtaining module, the first-round cut fragments $T_1$-$T_n$ are respectively compared with whole genome sequences of the remaining comparison strains by group iteration.

Optionally, when the first-round cut fragment $T_n$ is compared with whole genome sequences of the remaining comparison strains by group iteration, the candidate specific region obtaining module includes a comparison strain grouping submodule, a first-round candidate sequence library obtaining submodule, and a candidate specific region obtaining submodule.

The comparison strain grouping submodule divides the remaining comparison strains into P groups, each group includes a plurality of comparison strains.

The first-round candidate sequence library obtaining submodule simultaneously compares the first-round cut fragment $T_n$ with the whole genome sequences of the comparison strains in the first group one-to-one, and removes fragments of which the similarity exceeds a preset value, to obtain a plurality of residual fragments as the first-round candidate sequence library of the first-round cut fragment $T_n$.

The candidate specific region obtaining submodule simultaneously compares a previous-round candidate sequence library of the first-round cut fragment $T_n$ with whole genome sequences of the comparison strains in a next group one-to-one, and removes fragments of which the similarity exceeds the preset value, to obtain a plurality of residual fragments as a next-round candidate sequence library of the first-round cut fragment $T_n$. The candidate specific region obtaining submodule is repeated from the first-round candidate sequence library until a P-th-round candidate sequence library is obtained as a candidate specific sequence library of the first-round cut fragment $T_n$;

a collection of all the candidate specific sequence libraries of the first-round cut fragments is the candidate specific region.

The device further includes a candidate multi-copy region searching module and a multi-copy region verifying and obtaining module for obtaining multi-copy regions.

The candidate multi-copy region searching module performs internal alignment on a microorganism target fragment, and searches for a region corresponding to a to-be-detected sequence of which a similarity meets a preset value as a candidate multi-copy region, the similarity is a product of a coverage rate and a matching rate of the to-be-detected sequence.

The multi-copy region verifying and obtaining module obtains a median value of copy numbers of the candidate multi-copy region; if the median value of the copy numbers of the candidate multi-copy region is greater than 1, the candidate multi-copy region is recorded as a multi-copy region.

$$\text{The coverage rate=(length of similar sequence/(end value of the}$$

$$\text{to-be-detected sequence - starting value of the to-be-detected sequence + 1))\%}$$

When the microorganism target fragment includes multiple incomplete motifs, the motifs are connected together before searching for candidate multi-copy regions.

The multi-copy region verifying and obtaining module further includes a candidate multi-copy region copy number median value obtaining submodule, to determine the position of each candidate multi-copy region on the microorganism target fragment, obtain the number of other candidate multi-copy regions covering the position of each base of the to-be-verified candidate multi-copy region, and calculate the median value of the copy numbers of the to-be-verified candidate multi-copy region.

In an embodiment, the device further includes a final species-specific consensus sequence screening module, to obtain the candidate probes and primers by designing the probes and primers for the primary-screened species-specific consensus sequence according to the design rule of probes and primers. The sequence of the candidate probe and primer is aligned to the whole genome of all target strains, the strain coverage corresponding to the sequence of each probe and primer is calculated, the candidate probe and primer of which the strain coverage meets a preset value is screened out, and the primary-screened species-specific consensus sequence corresponding to the screened candidate probe and primer is taken as the final species-specific consensus sequence..

In an embodiment, the device further includes a first consensus sequence combination screening module. If none of the strain coverage rates of the candidate consensus sequences in the primary-screened species-specific consensus sequence verifying and obtaining module reaches the preset value, the first consensus sequence combination screening module combines the candidate consensus sequences, screens out a combination with a strain coverage rate reaching the preset value and having the least consensus sequence, takes the screened combination as the candidate consensus sequence, and verifies and obtains the primary-screened species-specific consensus sequences by the primary-screened species-specific consensus sequence verifying and obtaining module.

In an embodiment, the device further includes a second consensus sequence combination screening module. If none of the strain coverage rates of the candidate probes and primers in the final species-specific consensus sequence screening module reaches the preset value, the second consensus sequence combination screening module combines the primary-screened species-specific consensus sequences, screens out a combination with a strain coverage rate reaching the preset value and having the least consensus sequence, takes the screened combination as the candidate consensus sequence, and verifies and obtains the primary-screened species-specific consensus sequences by the primary-screened species-specific consensus sequence verifying and obtaining module.

In the first consensus sequence combination screening module and the second consensus sequence combination screening module, the combination may be performed according to the number of consensus sequences from low to high for selection.

In an embodiment, the device further includes a sequence update coverage rate module, to align the original candidate probes and primers to the updated whole genomes of the target strains when the number of target strains is updated, calculate the coverage rate, and verify whether the original candidate probes and primers can cover the updated target strains.

Users may submit the latest sequence data set through an interface. The sequence update coverage rate module may re-integrate the latest sequence data set into the database, to calculate the coverage rate by re-comparing the sequence of the original probes and primers to the updated sequence. The result may reflect whether the sequence of the original probes and primers can cover the newer strain.

Optionally, the multi-copy region verifying and obtaining module is further used to calculate a 95% confidence interval of the copy numbers of the candidate multi-copy region. preferably, when calculating the 95% confidence interval of the copy numbers of the candidate multi-copy region, a base number of the candidate multi-copy region serves as a sample number, and a copy number value corresponding to each base in the candidate multi-copy region serves as a sample value.

Since the principles of the device in the present embodiment is basically the same as that of the above-mentioned method embodiment, the definitions of the same features, the calculation methods, the enumeration of the embodiments, and the enumeration of the preferred embodiments may be used interchangeably, thus will not be described again.

It should be noted that the division of each module of the above apparatus is only a division of logical functions. In actual implementation, the modules may be integrated into one physical entity in whole or in part, or may be physically separated. These modules may all be implemented in the form of processing component calling by software. These modules may also be implemented entirely in hardware. It is also possible that some modules are implemented in the form of processing component calling by software, and some modules are implemented in the form of hardware. For example, the obtaining module may be a separate processing element, or may be integrated in a chip, or may be stored in a memory in the form of program code. The function of the above obtaining module is called and executed by one of the processing elements. The implementation of other modules is similar. In addition, all or part of these modules may be integrated or implemented independently. The processing elements described herein may be an integrated circuit with signal processing capabilities. In the implementation process, each operation of the above method or each of the above modules may be completed by an integrated logic circuit of hardware in the processor element or instruction in a form of software.

For example, the above modules may be one or more integrated circuits configured to implement the above method, such as one or more application specific integrated circuits (ASIC), or one or more digital signal processors (DSP), or one or more field programmable gate arrays (FPGA), or graphics processing unit (GPU). As another example, when one of the above modules is implemented in the form of calling program codes of a processing element, the processing element may be a general processor, such as a central processing unit (CPU) or other processors that may call program codes. As another example, these modules may be integrated and implemented in the form of a system-on-a-chip (SOC).

Some embodiments of the present disclosure further provide a computer readable storage medium, which stores a computer program. When executed by a processor, the program implements the above-mentioned method for identifying specific regions in microorganism target fragments.

Some embodiments of the present disclosure provide a computer processing device, including a processor and the above-mentioned computer readable storage medium. The processor executes the computer program on the computer readable storage medium to implement the operations of the above-mentioned method for identifying specific regions in microorganism target fragments.

Some embodiments of the present disclosure provide an electronic terminal, including a processor, a memory and a communicator; the memory stores a computer program, the communicator communicates with an external device, and the processor executes the computer program stored in the memory, so that the electronic terminal executes and implements the above-mentioned method for identifying specific regions in microorganism target fragments.

FIG. 3 is a schematic diagram showing the electronic terminal provided by the present disclosure. The electronic terminal includes a processor 31, a memory 32, a communicator 33, a communication interface 34 and a system bus 35; the memory 32 and the communication interface 34 are connected and communicated with the processor 31 and the communicator 33 through the system bus 35. The memory 32 is used to store computer programs. The communicator 33 and the communication interface 34 are used to communicate with other devices. The processor 31 and the communicator 33 are used to execute the computer program, so that the electronic terminal performs the operations of the above method for identifying specific regions in microorganism target fragments.

The system bus mentioned above may be a peripheral component interconnect (PCI) bus or an extended industry standard architecture (EISA) bus, etc. The system bus may include address bus, data bus, control bus and so on. For convenience of representation, only a thick line is used in the figure, but it does not mean that there is only one bus or one type of bus. The communication interface is used to implement communication between the database access device and other devices (such as a client, a read-write library, and a read-only library). The memory 301 may include a random access memory (RAM), or may also include a non-volatile memory, such as at least one disk memory.

The above-mentioned processor may be a general processor, including a central processing unit (CPU), a network processor (NP), and the like. The above-mentioned processor may also be a digital signal processor (DSP), an application specific integrated circuit (ASIC), a field-programmable gate array (FPGA), a graphics Processing unit (GPU) or other programmable logic devices, discrete gate or transistor logic devices, or discrete hardware components.

Those of ordinary skill will understand that all or part of the operations to implement the various method embodiments described above may be accomplished by hardware associated with a computer program. The computer program may be stored in a computer readable storage medium. The program, when executed, performs the operations including the above method embodiments. The computer readable storage mediums may include, but are not limited to, floppy disks, optical disks, compact disc read-only memories (CD-ROM), magneto-optical disks, read only memories (ROM), random access memories (RAM), erasable programmable read-only memory (EPROM), electrically erasable programmable read-only memory (EEPROM), magnetic cards or optical cards, flash memories, or other types of medium or machine-readable media suitable for storing machine-executable instructions. The computer readable storage medium may be a product that is not accessed to a computer device, or a component that has been accessed to a computer device for use.

In terms of specific implementation, the computer programs may be routines, programs, objects, components, data structures or the like that perform specific tasks or implement specific abstract data types.

The above-mentioned method for obtaining species-specific consensus sequences of microorganisms, the above-mentioned device for obtaining species-specific consensus sequences of microorganisms, the above-mentioned computer readable storage medium, the above-mentioned computer processing device or the above-mentioned electronic terminal may be used for screening template sequences in nucleotide amplification.

The screening is performed using species-specific consensus sequences as template sequences. The species-specific consensus sequences may be the primary-screened species-specific consensus sequences obtained by operation S200 or the primary-screened species-specific consensus sequence verifying and obtaining module, or the final species-specific consensus sequences obtained by operation S300 or the final species-specific consensus sequence screening module.

An embodiment of the present disclosure provides a method for identifying microbial species, which includes: identifying, by means of amplification, whether the target strain contains a species-specific consensus sequence obtained by the above-mentioned method.

The method of the present disclosure is capable of distinguishing whether the source strain of the microorganism target fragment and a comparison strain belong to the same species or subspecies.

The microorganism may include one or more of bacterium, virus, fungus, amoeba, cryptosporidium, flagellate, microsporidium, piroplasma, plasmodium, toxoplasma, trichomonas and kinetoplastid.

The above-mentioned embodiments are merely illustrative of the principle and effects of the present disclosure instead of limiting the present disclosure. Modifications or variations of the above-described embodiments may be made by those skilled in the art without departing from the spirit and scope of the disclosure. Therefore, all equivalent modifications or changes made by those who have common knowledge in the art without departing from the spirit and technical concept disclosed by the present disclosure shall be still covered by the claims of the present disclosure.

**Claims**

1. A method for obtaining species-specific consensus sequences of microorganisms, comprising at least:

S100, searching for candidate consensus sequences: clustering specific sequences of target strains belonging to the same species based on a clustering algorithm to obtain a plurality of candidate species-specific consensus sequences;

S200, verifying and obtaining primary-screened species-specific consensus sequences:

judging whether the candidate species-specific consensus sequences meet the following conditions:

1) a strain coverage rate meets a preset value;
2) an effective copy number meets a preset value;

if the candidate species-specific consensus sequences meet all the above conditions, determining that the candidate species-specific consensus sequences are species-specific consensus sequences;
wherein,

the strain coverage rate = (number of target strains with the candidate species-specific consensus sequence / total number of target strains) * 100%;

the effective copy number is calculated according to formula (I):

$$\sum_{i=0}^{n} Ci * (\frac{Si}{Sall})$$

(I);

wherein,

n is a total number of copy number gradients of the candidate species-specific consensus sequences;
Ci is the copy number corresponding to the i-th candidate species-specific consensus sequence;
Si is the number of strains with the i-th candidate species-specific consensus sequence;
Sail is a total number of the target strains.

2. The method for obtaining species-specific consensus sequences of microorganisms according to claim 1, wherein the specific sequences refer to target fragments belonging to the same target strain, and a region where the target fragments are located is a specific region of the target strain.

3. The method for obtaining species-specific consensus sequences of microorganisms according to claim 2, wherein the specific region is a specific multi-copy region.

4. The method for obtaining species-specific consensus sequences of microorganisms according to claim 2, wherein obtaining the specific region comprises:

S110, respectively comparing a microorganism target fragment with whole genome sequences of one or more comparison strains one-to-one, and removing fragments of which a similarity exceeds a preset value, to obtain a plurality of residual fragments as first-round cut fragments $T_1$-$T_n$, wherein n is an integer greater than or equal to 1;
S120, respectively comparing the first-round cut fragments $T_1$-$T_n$ with whole genome sequences of remaining comparison strains, and removing fragments of which a similarity exceeds the preset value, to obtain a collection of residual cut fragments as a candidate specific region of the microorganism target fragment; and
S130, verifying and obtaining the specific region: determining whether the candidate specific region meets the following requirements:

1) searching in public databases to find whether there are other species of which a similarity to the candidate specific region is greater than the preset value;
2) respectively comparing the candidate specific region with whole genome sequences of the comparison strains and a whole genome sequence of a host of a source strain of the microorganism target fragment, to find whether there are fragments with a similarity greater than the preset value; if the candidate specific region does not meet the above requirements, the candidate specific region is a specific region of the microorganism target fragment.

5. The method for obtaining species-specific consensus sequences of microorganisms according to claim 4, further comprising one or more of the followings:

a. the method is capable of distinguishing whether the source strain of the microorganism target fragment and a comparison strain belong to a same species or a same subspecies;
b.

b. the similarity refers to a product of a coverage rate and a matching rate of the microorganism target fragment, and the coverage rate = (length of similar sequence fragment/(end value of the microorganism target fragment - starting value of the microorganism target fragment + 1))%;

c. in operation S120, the first-round cut fragments $T_1$-$T_n$ are respectively compared with whole genome sequences of the remaining comparison strains by group iteration;
d. the preset value of similarity exceeds 80%;
e. positions of bases between two to-be-compared sequences do not cross;
f. the method further comprises: Sill, comparing selected adjacent microorganism target fragments one-to-one; if the similarity after comparison is lower than the preset value, issuing an alarm and displaying screening conditions corresponding to a target strain.

6. The method for obtaining species-specific consensus sequences of microorganisms according to claim 5, wherein the first-round cut fragment $T_n$ being compared with whole genome sequences of the remaining comparison strains by group iteration comprises:

S121, dividing the remaining comparison strains into P groups, each group including a plurality of comparison strains;

S122, simultaneously comparing the first-round cut fragment $T_n$ with the whole genome sequences of the comparison strains in the first group one-to-one, and removing fragments of which the similarity exceeds the preset value, to obtain a plurality of residual fragments as a first-round candidate sequence library of the first-round cut fragment $T_n$;

S123, simultaneously comparing a previous-round candidate sequence library of the first-round cut fragment $T_n$ with the whole genome sequences of the comparison strains in a next group one-to-one, and removing fragments of which a similarity exceeds the preset value, to obtain a plurality of residual fragments as a next-round candidate sequence library of the first-round cut fragment $T_n$; repeating operation S122 from the first-round candidate sequence library until a P-th-round candidate sequence library is obtained as the candidate specific sequence library of the first-round cut fragment $T_n$;

wherein a collection of all the candidate specific sequence libraries of the first-round cut fragments is the candidate specific region.

7. The method for obtaining species-specific consensus sequences of microorganisms according to claim 3, wherein obtaining the multi-copy region comprises:

S140, searching for a candidate multi-copy region: performing internal alignment on a microorganism target fragment, and searching for a region corresponding to a to-be-detected sequence of which a similarity meets a preset value as a candidate multi-copy region, the similarity being a product of a coverage rate and a matching rate of the to-be-detected sequence;

S150, verifying and obtaining a multi-copy region: obtaining a median value of copy numbers of the candidate multi-copy region; if the median value of the copy numbers of the candidate multi-copy region is greater than 1, the candidate multi-copy region is recorded as a multi-copy region.

8. The method for obtaining species-specific consensus sequences of microorganisms according to claim 7, further comprising one or more of the followings:

a.

a. the coverage rate = (length of similar sequence/(end value of the

to-be-detected sequence - starting value of the to-be-detected sequence +

$1))\%$;

b. when the microorganism target fragment includes multiple incomplete motifs, the motifs are connected together before searching for the candidate multi-copy region;

c. the obtaining of the median value of the copy numbers of the candidate multi-copy region includes: determining a position of each candidate multi-copy region on the microorganism target fragment, obtaining the number of other candidate multi-copy regions covering a position of each base of the to-be-verified candidate multi-copy region, and calculating the median value of the copy numbers of the to-be-verified candidate multi-copy region;

d. in operation S150, a 95% confidence interval of the copy numbers of the candidate multi-copy region is calculated; preferably, when calculating the 95% confidence interval of the copy numbers of the candidate multi-copy region, a base number of the candidate multi-copy region serves as a sample number, and a copy number value corresponding to each base in the candidate multi-copy region serves as a sample value.

9. The method for obtaining species-specific consensus sequences of microorganisms according to claim 1, further comprising one or more of the following operations:

S300, obtaining a candidate probes and primers by designing the probes and primers for the primary-screened species-specific consensus sequence according to a design rule of probes and primers; aligning a sequence of the candidate probes and primers to whole genomes of all target strains, calculating a strain coverage rate corresponding to the sequence of each probes and primers, screening out the candidate probes and primers of which the strain coverage rate meets a preset value, and taking a primary-screened species-specific consensus sequence corresponding to the screened candidate probes and primers as a final species-specific consensus sequence;

S400, if none of the strain coverage rates of the candidate consensus sequences in operation S200 reaches the preset value, combining the candidate consensus sequences, screening out a combination with a strain coverage rate reaching the preset value and having the least consensus sequence, taking the screened combination as the candidate consensus sequence, verifying and obtaining the primary-screened species-specific consensus sequences by S200.

**10.** The method for obtaining species-specific consensus sequences of microorganisms according to claim 9, further comprising:

S500, if none of the strain coverage rates of the candidate probes and primers in operation S300 reaches the preset value, combining the primary-screened species-specific consensus sequences, screening out a combination with a strain coverage rate reaching the preset value and having the least consensus sequence, taking the screened combination as the candidate consensus sequence, verifying and obtaining the primary-screened species-specific consensus sequences by S200.

**11.** The method for obtaining species-specific consensus sequences of microorganisms according to claim 9 or 10, wherein in operations S400 and S500, the combining is performed according to the number of consensus sequences from low to high for selection.

**12.** The method for obtaining species-specific consensus sequences of microorganisms according to claim 9 or 10, wherein when the number of target strains is updated, the original candidate probes and primers is aligned to updated whole genomes of the target strains, a coverage rate is calculated, and whether the original candidate probes and primers can cover the updated target strains is verified.

**13.** A device for obtaining species-specific consensus sequences of microorganisms, comprising:

a candidate consensus sequence searching module, configured to obtain a plurality of candidate species-specific consensus sequences by clustering specific sequences of target strains belonging to a same species based on a clustering algorithm;

a primary-screened species-specific consensus sequence verifying and obtaining module, configured to judge whether the candidate species-specific consensus sequences meet the following conditions:

1) a strain coverage rate meets a preset value;
2) an effective copy number meets a preset value;
if the candidate species-specific consensus sequences meet all the above conditions, determining that the candidate species-specific consensus sequences are species-specific consensus sequences;
wherein,

the strain coverage rate = (number of target strains with the candidate species-specific consensus sequence / total number of target strains) * 100%;

the effective copy number is calculated according to formula (I):

$$\sum_{i=0}^{n} Ci * \left(\frac{Si}{Sall}\right)$$
(I);

wherein,

n is a total number of copy number gradients of the candidate species-specific consensus sequences;

Ci is the copy number corresponding to the i-th candidate species-specific consensus sequence;

Si is the number of strains with the i-th candidate species-specific consensus sequence;

Sail is a total number of the target strains.

14. The device for obtaining species-specific consensus sequences of microorganisms according to claim 13, wherein the specific sequences refer to target fragments belonging to the same target strain, and a region where the target fragments are located is a specific region of the target strain.

15. The device for obtaining species-specific consensus sequences of microorganisms according to claim 14, wherein the specific region is a specific multi-copy region.

16. The device for obtaining species-specific consensus sequences of microorganisms according to claim 13, further comprising the following modules for obtaining a specific region:

a first-round cut fragment obtaining module, configured to respectively compare a microorganism target fragment with whole genome sequences of one or more comparison strains one-to-one, and remove fragments of which a similarity exceeds a preset value, to obtain a plurality of residual fragments as first-round cut fragments $T_1$-$T_n$, wherein n is an integer greater than or equal to 1;

a candidate specific region obtaining module, configured to respectively compare the first-round cut fragments $T_1$-$T_n$ with whole genome sequences of remaining comparison strains, and remove fragments of which the similarity exceeds the preset value, to obtain a collection of residual cut fragments as a candidate specific region of the microorganism target fragment; and

a specific region verifying and obtaining module, configured to determine whether the candidate specific region meets the following requirements:

1) public databases are searched in to find whether there are other species of which a similarity to the candidate specific region is greater than the preset value;

2) the candidate specific region is compared with whole genome sequences of the comparison strains and a whole genome sequence of a host of a source strain of the microorganism target fragment respectively, to find whether there are fragments with a similarity greater than the preset value;

if the candidate specific region does not meet the above requirements, the candidate specific region is a specific region of the microorganism target fragment.

17. The device for obtaining species-specific consensus sequences of microorganisms according to claim 16, further comprising one or more of the following:

a. the device is capable of distinguishing whether the source strain of the microorganism target fragment and a comparison strain belong to the same species or the same subspecies;

b.

b. the similarity refers to a product of a coverage rate and a matching rate of the microorganism target fragment, and the coverage rate = (length of similar sequence fragment/(end value of the microorganism target fragment - starting value of the microorganism target fragment + 1))%;

c. in the candidate specific region obtaining module, the first-round cut fragments $T_1$-$T_n$ are respectively compared with whole genome sequences of the remaining comparison strains by group iteration;

d. the preset value of similarity exceeds 80%;

e. positions of bases between two to-be-compared sequences do not cross;

f. the first-round cut fragment obtaining module further includes a raw data similarity comparison submodule, to compare selected adjacent microorganism target fragments one-to-one; if the similarity after comparison is lower than the preset value, an alarm is issued and the screening conditions corresponding to a target strain are displayed.

**18.** The device for obtaining species-specific consensus sequences of microorganisms according to claim 17, wherein when a first-round cut fragment $T_n$ is compared with whole genome sequences of the remaining comparison strains by group iteration, the candidate specific region obtaining module includes the following submodules:

a comparison strain grouping submodule, configured to divide the remaining comparison strains into P groups, each group including a plurality of comparison strains;

a first-round candidate sequence library obtaining submodule, configured to simultaneously compare the first-round cut fragment $T_n$ with the whole genome sequences of the comparison strains in the first group one-to-one, and remove fragments of which the similarity exceeds the preset value, to obtain a plurality of residual fragments as a first-round candidate sequence library of the first-round cut fragment $T_n$;

a candidate specific region obtaining submodule, configured to simultaneously compare a previous-round candidate sequence library of the first-round cut fragment $T_n$ with whole genome sequences of the comparison strains in a next group one-to-one, and remove fragments of which the similarity exceeds the preset value, to obtain a plurality of residual fragments as a next-round candidate sequence library of the first-round cut fragment $T_n$; the candidate specific region obtaining submodule is repeated from the first-round candidate sequence library until a P-th-round candidate sequence library is obtained as a candidate specific sequence library of the first-round cut fragment $T_n$;

wherein a collection of all the candidate specific sequence libraries of the first-round cut fragments is the candidate specific region.

**19.** The device for obtaining species-specific consensus sequences of microorganisms according to claim 15, further comprising the following modules for obtaining a multi-copy region:

a candidate multi-copy region searching module, configured to perform internal alignment on a microorganism target fragment, and search for a region corresponding to a to-be-detected sequence of which a similarity meets a preset value as a candidate multi-copy region, the similarity being a product of a coverage rate and a matching rate of the to-be-detected sequence;

a multi-copy region verifying and obtaining module, configured to obtain a median value of copy numbers of the candidate multi-copy region; if the median value of the copy numbers of the candidate multi-copy region is greater than 1, the candidate multi-copy region is recorded as a multi-copy region.

**20.** The device for obtaining species-specific consensus sequences of microorganisms according to claim 19, further comprising one or more of the following:

a.

a. the coverage rate = (length of similar sequence/(end value of the

to-be-detected sequence - starting value of the to-be-detected sequence +

$1))\%$;

b. when the microorganism target fragment includes multiple incomplete motifs, the motifs are connected together before searching for the candidate multi-copy region;

c. the multi-copy region verifying and obtaining module further includes a candidate multi-copy region copy number median value obtaining submodule, to determine a position of each candidate multi-copy region on the microorganism target fragment, obtain the number of other candidate multi-copy regions covering a position of each base of the to-be-verified candidate multi-copy region, and calculate the median value of the copy numbers of the to-be-verified candidate multi-copy region;

d. the multi-copy region verifying and obtaining module is further configured to calculate a 95% confidence interval of the copy numbers of the candidate multi-copy region; preferably, when calculating the 95% confidence interval of the copy numbers of the candidate multi-copy region, a base number of the candidate multi-copy region serves as a sample number, and a copy number value corresponding to each base in the candidate multi-copy region serves as a sample value.

21. The device for obtaining species-specific consensus sequences of microorganisms according to claim 13, further comprising one or more of the following modules:

a final species-specific consensus sequence screening module, configured to obtain a candidate probes and primers by designing the probes and primers for the primary-screened species-specific consensus sequence according to a design rule of probes and primers, align a sequence of the candidate probes and primers to whole genomes of all target strains, calculate a strain coverage rate corresponding to the sequence of each probes and primers, screen out the candidate probes and primers of which the strain coverage rate meets a preset value, and take a primary-screened species-specific consensus sequence corresponding to the screened candidate probes and primers as a final species-specific consensus sequence;
a first consensus sequence combination screening module, configured to combine the candidate consensus sequences, screen out a combination with a strain coverage rate reaching the preset value and having the least consensus sequence, take the screened combination as the candidate consensus sequence, and verify and obtain the primary-screened species-specific consensus sequences by the primary-screened species-specific consensus sequence verifying and obtaining module if none of the strain coverage rates of the candidate consensus sequences in the primary-screened species-specific consensus sequence verifying and obtaining module reaches the preset value.

22. The device for obtaining species-specific consensus sequences of microorganisms according to claim 21, further comprising:
a second consensus sequence combination screening module, configured to combine the primary-screened species-specific consensus sequences, screen out a combination with a strain coverage rate reaching the preset value and having the least consensus sequence, take the screened combination as the candidate consensus sequence, and verify and obtain the primary-screened species-specific consensus sequences by the primary-screened species-specific consensus sequence verifying and obtaining module if none of the strain coverage rates of the candidate probes and primers in the final species-specific consensus sequence screening module reaches the preset value.

23. The device for obtaining species-specific consensus sequences of microorganisms according to claim 21 or 22, wherein in the first consensus sequence combination screening module and the second consensus sequence combination screening module, the combining is performed according to the number of consensus sequences from low to high for selection.

24. The device for obtaining species-specific consensus sequences of microorganisms according to claim 21 or 22, further comprising:
a sequence update coverage rate module, configured to align an original candidate probes and primers to updated whole genomes of the target strains when the number of target strains is updated, calculate the coverage rate, and verify whether the original candidate probes and primers can cover the updated target strains.

25. A computer readable storage medium, which stores a computer program, wherein when executed by a processor, the program implements the method for obtaining species-specific consensus sequences of microorganisms according to any one of claims 1-12.

26. A computer processing device, comprising a processor and the computer readable storage medium according to claim 25, wherein the processor executes a computer program on the computer readable storage medium to implement operations of the method for obtaining species-specific consensus sequences of microorganisms according to any one of claims 1-12.

27. An electronic terminal, comprising a processor, a memory and a communicator; the memory stores a computer program, the communicator communicates with an external device, and the processor executes a computer program stored in the memory, so that the terminal executes the method for obtaining species-specific consensus sequences of microorganisms according to any one of claims 1-12.

28. A use of the method for obtaining species-specific consensus sequences of microorganisms according to any one of claims 1-12, the device for obtaining species-specific consensus sequences of microorganisms according to any one of claims 13-24, the computer readable storage medium according to claim 25, the computer processing device according to claim 26 or the electronic terminal according to claim 27 for screening template sequences in nucleotide amplification.

29. A method for identifying microbial species, comprising: identifying whether a target strain contains a species-specific consensus sequence by means of amplification, wherein the species-specific consensus sequence is obtained by the method for obtaining species-specific consensus sequences of microorganisms according to any one of claims 1-12, the device for obtaining species-specific consensus sequences of microorganisms according to any one of claims 13-24, the computer readable storage medium according to claim 25, the computer processing device according to claim 26 or the electronic terminal according to claim 27.

30. The method for identifying microbial species according to claim 29, further comprising one or more of the following:

    a. the method is capable of distinguishing whether a source strain of the microorganism target fragment and a comparison strain belong to the same species or the same subspecies;
    b. the microorganism includes one or more of bacterium, virus, fungus, amoeba, cryptosporidium, flagellate, microsporidium, piroplasma, plasmodium, toxoplasma, trichomonas and kinetoplastid.

Start

S100

Searching for candidate consensus sequences: clustering specific sequences of target strains belonging to a same species based on a clustering algorithm, to obtain a plurality of candidate species-specific consensus sequences

S200

Verifying and obtaining primary-screened species-specific consensus sequences: judging whether the candidate species-specific consensus sequences meet the following conditions:

1) a strain coverage rate meets a preset value;

2) an effective copy number meets a preset value;

if the candidate species-specific consensus sequences meet all the above conditions, determining that the candidate species-specific consensus sequences are species-specific consensus sequences

End

FIG. 1

```
ClusterID       Distribution        Marker  Percent_of_strains      Weighted_Average_Copy
Cluster43       9(5)|      *        100.0%[5/5]       9
Cluster226      7(1)|8(2)|9(2)|     *        100.0%[5/5]      8.2
Cluster56       8(5)|      *        100.0%[5/5]       8
Cluster36       7(1)|8(3)|9(1)|     *        100.0%[5/5]      8
Cluster123      5(1)|8(3)|9(1)|     *        100.0%[5/5]      7.6
Cluster5        5(1)|7(1)|8(3)|     *        100.0%[5/5]      7.2
Cluster39       6(2)|7(2)|9(1)|     *        100.0%[5/5]      7
Cluster194      6(1)|7(3)|8(1)|     *        100.0%[5/5]      7
Cluster67       6(2)|7(2)|8(1)|     *        100.0%[5/5]      6.8
Cluster10       4(1)|7(2)|8(2)|     *        100.0%[5/5]      6.8
Cluster182      3(1)|6(1)|7(2)|8(1)|        *        100.0%[5/5]      6.2
Cluster52       4(1)|5(1)|6(1)|7(1)|8(1)|          *        100.0%[5/5]      6
Cluster249      3(1)|6(2)|7(1)|8(1)|        *        100.0%[5/5]      6
Cluster47       5(1)|6(4)|         *        100.0%[5/5]      5.8
Cluster186      3(1)|5(1)|6(1)|7(1)|8(1)|          *        100.0%[5/5]      5.8
Cluster217      3(1)|5(2)|6(1)|7(1)|        *        100.0%[5/5]      5.2
Cluster64       3(1)|4(1)|6(3)|     *        100.0%[5/5]      5
Cluster94       1(1)|2(1)|5(1)|8(2)|        *        100.0%[5/5]      4.8
Cluster214      1(1)|3(1)|6(2)|8(1)|        *        100.0%[5/5]      4.8
Cluster148      2(1)|4(1)|5(2)|7(1)|        *        100.0%[5/5]      4.6
Cluster93       1(1)|3(1)|5(2)|8(1)|        *        100.0%[5/5]      4.4
Cluster234      0(1)|3(1)|6(2)|7(1)|        *        80.0%[4/5]      4.4
Cluster66       3(1)|4(2)|5(2)|     *        100.0%[5/5]      4.2
Cluster252      1(1)|4(1)|5(2)|6(1)|        *        100.0%[5/5]      4.2
Cluster24       3(2)|4(1)|5(1)|6(1)|        *        100.0%[5/5]      4.2
Cluster114      0(1)|3(1)|4(1)|7(2)|        *        80.0%[4/5]      4.2
Cluster203      0(1)|3(1)|5(2)|7(1)|        *        80.0%[4/5]      4
Cluster62       2(1)|3(1)|4(1)|5(2)|        *        100.0%[5/5]      3.8
Cluster46       2(1)|3(1)|4(1)|5(2)|        *        100.0%[5/5]      3.8
Cluster32       3(1)|4(4)|        *        100.0%[5/5]      3.8
Cluster235      1(1)|3(1)|5(3)|    *        100.0%[5/5]      3.8
Cluster205      3(1)|4(4)|        *        100.0%[5/5]      3.8
Cluster201      1(1)|3(1)|4(1)|5(1)|6(1)|          *        100.0%[5/5]      3.8
Cluster107      1(1)|4(2)|5(2)|    *        100.0%[5/5]      3.8
Cluster2        1(1)|3(1)|4(2)|6(1)|        *        100.0%[5/5]      3.6
Cluster141      0(1)|3(1)|4(2)|7(1)|        *        80.0%[4/5]      3.6
Cluster87       2(2)|3(1)|5(2)|    *        100.0%[5/5]      3.4
Cluster72       3(3)|4(2)|        *        100.0%[5/5]      3.4
Cluster246      3(3)|4(2)|        *        100.0%[5/5]      3.4
```

FIG. 1-1

S1  Target strain  `-----TCTCCCCCACGCTTTCCCGTTTCTCCACCGGGTTCAACCTCGCGGGGACTGTTTTGGACCAGGTGTTGGATTATGTTCCCTGGATTGGGAATGGGTACAGGTATGGGAATGACCACCGGGGGAGCA------`

Comparison strain 1  `-----CGACGTTTTCGCTTTCCCGTTTCTCCACCAAAGCCAACGTCCAAGCGACGCTCGGGGGCAGTCAGACGACGACTACCAAGGGTTGGGAATGGGTACAGTCCTCGACCCAGCCAACCTCCAGCTCTGAACCGGCGCGGGGTGAAGG------`

`-----TCTCCCCCACG`  `CGGGTTCAACCTCGCGGGGACTGTTTTGGACCAGGTGTTGGATTATGTTCCCTGGA`  `GTATGGGAATGACCACCGGGGGAGCA------`  `------------------`  `------------------`

Comparison strain 2  `-----AAGGGTTCGCCTTGAAGAATCCTCGACCTCGCGGGGATCCAGCTCTGAACCGGCGCGGGGTGAAGGAATGATAAGGCTTCATGGGAACAAAGTGACGAAAACCACACCACCTTTAAAAGCGCTACGGGGATGGGCAAGCAGGGA------`

S2

`-----TCTCCCCCACG`  `CTGTTTTGGACCAGGTGTTGGATTATGTTCCCTGGA`  `ATGACCACCGGGGGAGCA------`  `------------------`  `------------------`

Comparison strain 3  `-----CTCGACCCAGCCAACCTCCAGCTCTGAACCGGCGCGGGGTGAAGGAATGATAAGGCTATGACCACCGGGGGAGCAGAAAACCACACCACCTTTAAAAGCGCTACGGGG------`

N times of comparison

Specificity  `CTGTTTTGGACCAGGTGTTGGATTATGTTCCCTGGA`

`CTAAATTTTTAGCTTTCTCCCCCACG`

`------------------`

`------------------`

FIG. 1-2

| Sequence 1 | Sequence 2 | Sequence matching rate | Alignment length | Mismatch length | Length of gap openings | Starting value of sequence 1 | End value of sequence 1 | Starting value of sequence 2 | End value of sequence 2 | e value | Score |
|---|---|---|---|---|---|---|---|---|---|---|---|
| Query id | Subject id | % identity | alignment length | mismatches | gap openings | q. start | q. end | s. start | s. end | e-value | bit score |
| A | B | 98.4 | 187 | 3 | 0 | 1 | 187 | 1170 | 1356 | 1.90E-98 | 356 |
| | | | | | | | | | | | |
| Sequence coverage rate = (alignment length /(end value of sequence 1- starting value of sequence 1 + 1)) | | | | | | | | | | | |
| Similarity = sequence coverage rate * sequence matching rate | | | | | | | | | | | |
| | | | | | | | | | | | |

FIG. 1-3

FIG. 1-4

FIG. 1-5

21

Candidate consensus
sequence searching
module

22

Primary-screened
species-specific
consensus sequence
verifying and obtaining
module

FIG. 2

33

Communicator

31

Processor

35

34

Communication
interface

32

Memory

FIG. 3

## INTERNATIONAL SEARCH REPORT

| | International application No. |
| --- | --- |
| | **PCT/CN2020/090177** |

| | |
| --- | --- |
| **A.    CLASSIFICATION OF SUBJECT MATTER** | |
| G16B 30/10(2019.01)i;   G16B 40/10(2019.01)i | |
| According to International Patent Classification (IPC) or to both national classification and IPC | |

| | |
| --- | --- |
| **B.      FIELDS SEARCHED** | |
| Minimum documentation searched (classification system followed by classification symbols) | |
| G16B | |
| Documentation searched other than minimum documentation to the extent that such documents are included in the fields searched | |
| Electronic data base consulted during the international search (name of data base and, where practicable, search terms used) | |
| CNABS, CNTXT, SIPOABS, DWPI, VEN, WOTXT, USTXT, EPTXT, CNKI, 万方数据库, baidu学术, PUBMED, ELSEVIER, SPRINGER, ISI web of knowledge, sciencedirect: 之江生物, 嵇匀, 邵俊斌, 刘燕, 齐霞, 金宇丹, 李启腾, 微生物, 菌种, 菌株, 细菌, 病毒, 真菌, 变形虫, 隐孢子虫, 鞭毛虫, 微孢子虫, 梨形虫, 疟原虫, 弓形虫, 毛滴虫, 动质体, 种, 特异性共有序列, 覆盖度, 有效拷贝数, 聚类, 迭代, microbial, bacteria, microorganism, virus, fungi, amoeba, cryptosporidium, flagellate, microsporidium, pear-shaped insect, plasmodium, moving body, species, specific consensus sequence, specific consequence, unique, idiotype, coverage value, effective copy number, clustering, iteration, zj bio-tech, cong ji, junbin shao, yan liu, xia qi, yudan jin, qiteng li | |

| | |
| --- | --- |
| **C.      DOCUMENTS CONSIDERED TO BE RELEVANT** | |

| Category* | Citation of document, with indication, where appropriate, of the relevant passages | Relevant to claim No. |
| --- | --- | --- |
| A | CN 103714267 A (INSTITUTE OF BIOTECHNOLOGY, THE ACADEMY OF MILITARY MEDICAL SCIENCES, CHINA) 09 April 2014 (2014-04-09)<br>entire document | 1-30 |
| A | CN 110111843 A (BGI TECH SOLUTIONS (SHENZHEN) CO., LTD.) 09 August 2019 (2019-08-09)<br>entire document | 1-30 |
| A | CN 109949867 A (INSTITUTE OF SPECIAL ANIMAL AND PLANT SCIENCES OF CAAS) 28 June 2019 (2019-06-28)<br>entire document | 1-30 |
| A | CN 110875082 A (SHENZHEN BGI YINYUAN PHARMACEUTICAL TECHNOLOGY CO., LTD.) 10 March 2020 (2020-03-10)<br>entire document | 1-30 |

| | | |
| --- | --- | --- |
| ✓ Further documents are listed in the continuation of Box C. | ✓ See patent family annex. | |

| | |
| --- | --- |
| *      Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **12 December 2020** | **31 December 2020** |

| Name and mailing address of the ISA/CN | Authorized officer |
| --- | --- |
| **China National Intellectual Property Administration (ISA/ CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2020/090177** |

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2014/144529 A1 (COSMOSID INC.) 18 September 2014 (2014-09-18)<br>        entire document | 1-30 |
| A | WO 2010/016071 A2 (SWATI, SUBODH) 11 February 2010 (2010-02-11)<br>        entire document | 1-30 |
| A | VETROVSKY, T.et al. "The Variability of the 16S rRNA Gene in Bacterial Genomes and Its<br>Consequences for Bacterial Community Analyses"<br>*PLOS ONE,* Vol. 8, No. 2, 27 February 2013 (2013-02-27),<br>        e57923, pages 1-10 | 1-30 |
| PA | CN 110970093 A (SHENZHEN BGI YINYUAN PHARMACEUTICAL TECHNOLOGY<br>CO., LTD.) 07 April 2020 (2020-04-07)<br>        entire document | 1-30 |

Form PCT/ISA/210 (second sheet) (January 2015)

**INTERNATIONAL SEARCH REPORT**
**Information on patent family members**

International application No.

**PCT/CN2020/090177**

| Patent document cited in search report | | | Publication date (day/month/year) | Patent family member(s) | | | Publication date (day/month/year) |
|---|---|---|---|---|---|---|---|
| CN | 103714267 | A | 09 April 2014 | CN | 103714267 | B | 17 August 2016 |
| CN | 110111843 | A | 09 August 2019 | None | | | |
| CN | 109949867 | A | 28 June 2019 | None | | | |
| CN | 110875082 | A | 10 March 2020 | None | | | |
| WO | 2014/144529 | A1 | 18 September 2014 | EP | 2972309 | A1 | 20 January 2016 |
| | | | | US | 2014288844 | A1 | 25 September 2014 |
| | | | | JP | 6644672 | B2 | 12 February 2020 |
| | | | | EP | 2972309 | A4 | 23 November 2016 |
| | | | | CA | 2906725 | A1 | 18 September 2014 |
| | | | | JP | 2016518822 | A | 30 June 2016 |
| WO | 2010/016071 | A2 | 11 February 2010 | WO | 2010016071 | A3 | 29 July 2010 |
| CN | 110970093 | A | 07 April 2020 | None | | | |

Form PCT/ISA/210 (patent family annex) (January 2015)